# FASCICULE DE BREVET EUROPEEN

(11) **EP 0 742 231 B1**
(45) Date de publication et mention de la délivrance du brevet: **28.06.2000**
(21) Numéro de dépôt: 96400755.3
(22) Date de dépôt: 05.04.1996
(51) Int. Cl.: C08F 2/32, C08F 265/02, C08J 3/12

(54) **Procédé pour l'obtention de polymères superabsorbants pour l'eau et les fluides aqueux sous forme d'agrégats de particules**
Verfahren zur Herstellung von Wasser oder wässrige Flüssigkeiten absorbierende Polymeren in Form von aggregierten Partikeln
Process for obtaining superabsorbent polymers for water and aqueous fluids as aggregates of particles

(30) Priorité: 11.04.1995 FR 9504325
(43) Date de publication de la demande: 13.11.1996
(73) Titulaire: ELF ATOCHEM S.A., 92800 Puteaux, Hauts-de-Seine (FR)
(72) Inventeur: Collette, Christian, 75005 Paris (FR); Hidalgo, Manuel, 75017 Paris (FR); Kowalik, André, 60270 Gouvieux (FR); Puchois, Emmanuel, 75019 Paris (FR); Rebre, Shu-Rong, 94300 Vincennes (FR)
(74) Mandataire: Haicour, Philippe

(56) Documents cités:
- EP-A- 0 036 463
- EP-A- 0 441 507
- EP-A- 0 522 570
- WO-A-90/08789

## Description

La présente invention concerne la production de résines polymères à haut pouvoir d'absorption d'eau, en particulier, des résines absorbantes sous la forme de particules sphériques agglomérées.

De telles résines sont particulièrement appréciées dans la fabrication de produits sanitaires, dont les couches jetables pour bébés, les serviettes hygiéniques et produits semblables. Elles sont également utiles dans d'autres secteurs d'applications, par exemple en agriculture, comme conditionneurs de sols ou comme agents de rétention d'eau, ou encore dans l'ingénierie civile, comme composants anticondensation ou déshydratants de certains matériaux de construction.

Le choix d'une résine absorbante appropriée implique la prise en compte d'un certain nombre de propriétés désirables, comme une forte absorbance, une bonne compatibilité avec les autres composants des produits sanitaires, une faible tendance au remouillage, une bonne force mécanique du gel gonflé par l'eau, qui sont des propriétés plutôt attachées à la nature chimique du polymère, et aussi des tailles des particules comprises entre 100 et 1.000 micromètres, avec distribution étroite de ces tailles et en particulier le moins possible de particules de petite taille, ceci pour des raisons de manipulation et de construction des articles qui les incorporent, une forte vitesse d'absorption, une bonne diffusion des liquides dans, entre et à l'intérieur des particules absorbantes, qui sont des caractéristiques qui tiennent à la granulométrie et à la forme des particules. En plus, une bonne résine absorbante d'eau doit encore être non irritante et bon marché.

Parmi les système absorbants d'eau les plus connus, on trouve les acides polyacryliques partiellement neutralisés et leurs dérivés auxquels l'invention fait recours. Traditionnellement, les résines superabsorbantes acryliques, qui sont parmi les objets essentiels de la présente invention, ont été produites essentiellement selon deux modes différents de polymérisation : la polymérisation en solution qui délivre des masses polymères qu'il faut ensuite rebroyer et calibrer pour obtenir une poudre de particules de granulométrie convenable, et la polymérisation en suspension inverse, dont le résultat est directement une poudre dont les éléments sont des particules plus ou moins sphériques de polymère.

Ce dernier mode d'obtention, qui est celui qui concerne la présente invention, présente toutefois le désavantage de délivrer en l'absence d'agglomération un polymère superabsorbant sous la forme d'une poudre de trop fine granulométrie. Il en est ainsi par exemple avec les procédés de polymérisation d'un sel alcalin d'acide acrylique ou d'un monomère voisin en suspension inverse eau dans l'huile tels que les décrivent la demande japonaise n° 54-30710 qui fait recours à un surfactant primaire constitué d'un ester d'acide gras et de sorbitan d'HLB compris entre 3 et 6 ou la demande japonaise No 60-25045 qui met en oeuvre un surfactant anionique, qui délivrent des poudres dont les grains ont des tailles comprises entre 10 et 100 micromètres. L'agglomération à part des particules individuelles initiales est une réponse connue aux besoins de disposer de particules finales à la fois de grande taille et avec la forte surface spécifique sans laquelle la vitesse d'absorption de l'eau serait insuffisante. Un certain nombre de méthodes d'agglomération ont été proposées. On trouve ainsi l'agglomération de poudres sèches, par réticulation de surface avec un agent de réticulation (EP 349240, Nippon Shokubai), ou par une solution aqueuse de monomère contenant un agent de réticulation (JP 1126314, Nippon Shokubai), ce qui a pour effet d'améliorer la vitesse d'absorption du polymère, mais c'est une opération techniquement délicate, qui a aussi l'inconvénient de délivrer des agglomérats relativement fragiles.

D'autres ont donc préféré la réagglomération d'une poudre remise en suspension dans un liquide hydrocarboné inerte à l'aide d'un agglomérant constitué d'une poudre minérale (silice) et d'un tensioactif (EP 224923 Seitetsu) ou d'une relativement faible quantité d'une solution aqueuse acrylique introduite dans la suspension de polymère sous conditions de polymérisation (WO 90/08789, Dow Chemicals).

Ces procédés ne constituent que des méthodes pour reprendre des poudres insatisfaisantes quant à leur granulométrie. C'est, au passage, une solution industriellement peu satisfaisante que d'avoir systématiquement à réaliser deux opérations complètes indépendantes pour un seul résultat. C'est pour cela que d'autres ont intégré la phase d'agglomération dans le procédé global de production du polymère superabsorbant en faisant suivre directement la polymérisation en suspension inverse qui délivre le polymère sous forme de particules individuelles de l'étape d'agglomération. Ainsi, Mitsubishi (EP 522570) introduit directement du monomère dans le slurry de première polymérisation, dont il provoque ensuite la polymérisation en portant le milieu à environ 60°C. La méthode fonctionne apparemment bien, mais pour des quantités limitées de monomère. Sumitomo (EP 441507) décrit également un procédé d'obtention en deux étapes, dans lequel après avoir opéré la première étape de polymérisation en suspension inverse, on refroidit à température ambiante la suspension de particules polymères de façon à inactiver le surfactant ayant assuré la stabilité de la suspension inverse; après quoi on ajoute une seconde charge de monomère, sous forme d'une solution aqueuse à la suspension, dont on provoque la polymérisation par élévation de température vers 60/70°C. On obtient ainsi une distribution étroite d'agglomérats de grande taille fortement liés, avec toutefois l'inconvénient d'une forte consommation d'énergie et d'une mauvaise productivité du fait de la lenteur des cycles nécessaires de refroidissement / réchauffement du réacteur.

La présente invention remédie à ces inconvénients en proposant un procédé d'obtention de polymère acrylique superabsorbant sous la forme d'une poudre d'agglomérats de particules sphériques par agglomération de particules fines de polymère superabsorbant à l'aide d'un agglomérant constitué par une solution aqueuse de monomère insaturé hydrosoluble, en suspension dans un milieu liquide hydrocarboné non-solvant pour le polymère et pour l'agglomérant, avec la condition que soient respectées ensemble les conditions suivantes :
- la solution aqueuse de monomère est introduite dans le milieu à la température de polymérisation du monomère, généralement à une température *comprise entre 60 et 75°C,*
- la quantité de monomère agglomérant est comprise entre 50 % et 200 % en poids du polymère à agglomérer,
- la solution aqueuse de monomère contient en outre un additif hydrophile constitué d'un épaississant et d'un tensioactif d'HLB supérieur ou égal à 8, ainsi qu'un amorceur de polymérisation.

Au sens de la présente invention, on entend par température de polymérisation, une température à laquelle s'opère *convenablement* la polymérisation du monomère, et qui dépend entre autres de l'amorceur de polymérisation utilisé. Dans le cas où cet amorceur est le persulfate de potassium, qui est un amorceur préféré pour l'invention, cette température est supérieure à 50°C.

Pour la réalisation de l'invention, on part soit d'une poudre fine de polymère superabsorbant que l'on disperse à chaud sous agitation modérée dans un milieu liquide hydrocarboné, soit directement de la bouillie de polymère en l'état de composition et de température qui résulte d'une polymérisation préalable en suspension inverse. On introduit ensuite sous agitation, et en maintenant au mieux la température du réacteur à la température de polymérisation, en pratique entre 60 et 75°C, la solution aqueuse de monomère hydrosoluble insaturé qui aura été préparée à part. Pour cette préparation, effectuée à froid pour éviter à ce stade toute polymérisation intempestive, on procède par dissolution et neutralisation partielle du monomère, addition d'initiateur de polymérisation le cas échéant d'agent de réticulation, ainsi que d'un additif hydrophile destiné à contrôler la formation directe de grappes de particules de polymères qui constituera la poudre de résine superabsorbante dans sa forme finale. La vitesse d'introduction de la solution de monomère et l'agitation dans le réacteur sont contrôlées de façon à ce que la température au sein du réacteur s'écarte le moins possible de la température de consigne choisie, l'addition de monomère froid ayant tendance à l'abaisser, et l'exothermicité de la polymérisation à l'augmenter. Lorsque la polymérisation est terminée, ce dont on est assuré lorsque la température du milieu est redevenue constante après passage du pic exothermique, l'eau et le solvant sont évacués par distillation et la poudre de résine superabsorbante est recueillie directement dans sa forme d'utilisation.

*Selon une autre forme du procédé le polymère superabsorbant est gonflé par de l'eau préalablement à sa mise en suspension dans le liquide hydrocarboné.*

Les monomères insaturés, les initiateurs de polymérisation, les agents de réticulation, les liquides hydrocarbonés non réactifs constituant le milieu dispersif, les agents tensioactifs, les épaississants qui interviennent dans l'invention sont des produits qu'on retrouve fréquemment dans les procédés de polymérisation radicalaire en milieu dispersé.

L'acide acrylique et l'acide méthacrylique sont des monomères insaturés hydrosolubles préférés de l'invention. Le procédé peut néanmoins être mis en oeuvre avec d'autres monomères hydrosolubles insaturés comme les amides méthylpropane-sulfoniques de ces acides, leurs dérivés non ioniques tels que l'acrylamide, le méthacrylamide et leur dérivés N,N-diméthyl substitués, le 2-hydroxyéthyl-acrylate ou méthacrylate, le N-méthylol-acrylamide ou méthacrylamide, ou encore leur dérivés azotés tels que le (diméthyl- ou diéthyl-)amino(éthyl ou propyl)-acrylate ou -méthylacrylate et les sels d'ammonium quaternaires correspondants.

La polymérisation est initiée par les initiateurs de polymérisation radicalaire, qu'on préfère hydrosolubles puisque aussi bien le monomère que le polymère sont eux-mêmes hydrophiles ; le persulfate de potassium est particulièrement adapté au cas présent.

Des agents de réticulation utiles pour la réticulation partielle des polymères hydrophiles produits, sont généralement constitués par des composés possédant au moins deux groupes insaturés copolymérisables avec le monomère insaturé (acide acrylique) dont le type est celui des di/triacrylates de polyols, ou susceptibles de réagir avec ses produits de polymérisation, comme les diglycidyléthers de diols.

Les liquides hydrocarbonés non réactifs utilisés pour l'invention comrpe milieux dispersifs sont des produits chimiquement inertes vis à vis des monomères et des polymères formés. Il est très souhaitable qu'ils forment un azéotrope avec l'eau pour que celle-ci puisse être éliminée par distillation azéotropique, et que leur point d'ébullition soit suffisamment peu élevé pour qu'ils puissent être évaporés de la poudre finale sans dégradation thermique. Ce sont par exemple le n-pentane, le cyclohexane, le n-heptane, le toluène, l'éthylcyclohexane, l'iso-octane, le xylène.

Il arrive que dans des conditions proches de celles de l'invention, il se produise une agglomération spontanée des particules de polymère. On ne peut évidemment pas compter sur un tel processus pour obtenir le résultat industriel que l'on recherche. On atteint ici ce résultat en ajoutant à l'agglomérant monomère un agent très hydrophile à propriété surfactive et viscosante. Cet agent hydrophile est constitué par l'association d'un surfactant hydrophile d'HLB supérieur ou égal à 8 et d'un agent viscosant ou épaississant. Comme surfactant hydrophile, on peut utiliser par exemple des agents non ioniques comme les alkylphénols éthoxylés, le sorbitane ou les dérivés de sorbitol éthoxylés ; parmi ceux-ci on apprécie particulièrement ceux qui contiennent 10 à 100 molécules, et plus précisément 15 à 50 molécules d'oxyde d'éthylène par molécule; ou des tensioactifs ioniques conventionnels tels que les sulfates ou les sulfonates, par exemple le sodium dodécylsulfate ou l'acide dodécylbenzènesulfonique; des agents tensioactifs polymériques hydrophiles comme l'alcool polyvinylique ou les copolymères acétate de vinyle / alcool vinylique. Le choix des agents viscosants ou épaississants hydrophiles peut se porter sur les dérivés de la cellulose dits celluloses hydrosolubles modifiées, tels que la carboxyméthylcellulose, ou encore sur des polymères acryliques hydrosolubles comme les acides poly(méth)acryliques linéaires ou leurs copolymères, par exemple avec l'acrylate de 2-hydroxyéthyle, la vinylpyrrolidone ou ses dérivés, les copolymères linéaires acide (méth)acrylique / méthylvinyléther / acide ou anhydride maléique, leurs sels métalliques, les polyoxydes d'éthylène, les polyuréthanes hydrosolubles. On remarquera que l'utilisation d'un agent épaississant ou viscosant dans la charge de l'agglomérant va à l'encontre d'un préjugé de l'art antérieur. Ce que l'on souhaite d'un agglomérant, quel que soit le mécanisme par lequel il se consomme, est qu'il vienne se fixer sur ou dans les particules qu'il a pour fonction d'agglomérer et non pas qu'il se mette en suspension stable et se polymérise sous cette forme, ce qui aurait pour résultat la formation d'une nouvelle population de particules individuelles de polymère, ce que l'on veut évidemment éviter. C'est l'un des enseignements inattendus de l'invention que non seulement un agent épaississant dans la charge de l'agglomérant ne favorise pas sa stabilisation sous forme de suspension au détriment de son contact avec les particules de polymères à agglomérer, mais qu'il soit une des caractéristiques du procédé.

*De manière préférentielle l'additif hydrophile est constitué d'un nonyl phénol éthoxylé à 50 moles d'oxyde d'éthylène comme tensio-actif à HLB supérieur à* 8 et *de la carboxyméthylcellulose comme épaississant.*

Le rapport pondéral entre l'agglomérant et le polymère à agglomérer n'est pas indifférent. En dessous de 50 % d'agglomérant, l'agglomération n'est que très partielle et la poudre finale contient une trop forte proportion de billes fines restées indépendantes. Au-dessus de 200 %, le processus souffre de difficultés de stabilisation : il y a trop de phase dispersée dans l'hydrocarbure qui constitue le milieu de dispersion, et il a risque sérieux de prise en masse.

Les exemples qui suivent feront mieux comprendre l'invention.

### Exemple 1 : préparation de particules de superabsorbant sans agglomération (exemple de comparaison).

Il s'agit du procédé de base de polymérisation en suspension inverse en un temps. On y distingue :
- une séquence a de préparation du milieu dispersif,
- une séquence b de préparation de la solution de monomère,
- une séquence c d'introduction et de dispersion du monomère dans le milieu dispersif et sa polymérisation,
- une séquence f (finale) d'évaporation de l'eau et du solvant et de récupération de la poudre obtenue.

### Séquence a)

Dans un réacteur d'un litre muni d'un dispositif d'introduction de réactifs solides ou liquides, d'un agitateur à pales, d'un système de balayage par gaz neutre, d'une sonde de température et d'un dispositif de chauffage/réfrigération constitué par une double enveloppe dans laquelle circule un fluide caloporteur assignable à une température de consigne, on dissout vers 80°C et sous agitation de 400 tours/minute et balayage d'azote, 1,07 gramme de polyéthylène modifié par l'anhydride maléique (Hi-Wax 1105A, commercialisé sous ce nom par Mitsui Petrochemical Industries Co) et 0,55 gramme de di /tristéarate de saccharose dans 275 grammes d'heptane. Cette solution est maintenue à 80°C le temps nécessaire à la dissolution complète du système tensioactif dans l'heptane. Après dissolution, la température est ramenée à 60°C.

### Séquence b)

Dans un autre réacteur d'un litre équipé comme le précédent, on neutralise 92 grammes d'une solution aqueuse à 80 % en poids d'acide acrylique par 135,59 grammes de lessive de soude à 22,62 %, en procédant suffisamment lentement pour ne pas dépasser 30°C. A cette solution, on ajoute 2 grammes de carboxyméthylcellulose, puis 0,414 gramme d'une solution à 2 % en poids d'éthylèneglycoldiglycidyléther, ainsi que 2,76 grammes d'une solution aqueuse à 2 % de persulfate de potassium.

### Séquence c)

Le réacteur avec la solution à base d'heptane étant maintenu sous agitation de 400 tours/minute et sous balayage d'azote, on y introduit rapidement la phase aqueuse précédemment préparée qui passe en suspension inverse dans l'heptane. L'introduction rapide de la phase aqueuse dans le réacteur provoque une chute de la température du milieu vers 40°C. On maintient cette température pendant une période de stabilisation de 15 minutes. Après ce délai, la consigne de température du fluide caloporteur circulant dans la double enveloppe est portée à 70°C. La polymérisation démarre. On maintient le réacteur à ce niveau de température pendant 30 minutes.

### Séquence f) finale

On monte maintenant la température de consigne à 115-130°C pour éliminer l'eau et l'heptane. Après évaporation complète, on obtient un polymère superabsorbant sous forme d'une poudre constituée de billes individuelles de polymère superabsorbant d'une taille moyenne d'environ 100 µm, dont le pouvoir absorbant d'eau salée à 0,9 % en poids est de 75 grammes par gramme de polymère. Le pourcentage de fines (particules de taille inférieure à 100 µm) est d'environ 50 %, valeur tout à fait inacceptable.

### Exemples 2 à 7 sont des exemples de procédés comportant une étape d'agglomération de particules fines individuelles. On y distingue :

- les séquences a, b, c, de préparation du polymère superabsorbant en billes individuelles, qui peuvent être extemporanées, et sont alors enchaînés directement avec les séquences suivantes d, e f, ou bien qui correspondent à une préparation préliminaire de poudre sèche, auquel cas, elles ont été suivies d'une séquence f d'isolement de la poudre,
- une séquence d de préparation d'une charge d'agglomérant,
- une séquence e d'introduction de la charge agglomérante dans la poudre de billes individuelles de polymère ou dans un milieu dispersant au sein duquel ces billes restent en suspension,
- une seconde séquence f d'évaporation des solvants et de récupération de la poudre obtenue.

### Exemple 2 : réagglomération de poudre selon l'invention

### Séquence a)

On charge un réacteur tel que décrit à la séquence a de l'exemple précédent, avec 275 g d'heptane. La température du fluide caloporteur est consignée à la température de 80°C et sous agitation de 400 tours/minute et balayage d'azote, on y dissout 1,07 gramme de Hi-Wax 1105A et 0,55 gramme de di / tristéarate de saccharose. Cette solution est maintenue à 80°C le temps nécessaire à la dissolution du système dispersant dans l'heptane. La température est maintenue à 80°C, et on introduit dans le milieu, toujours sous agitation, la poudre de superabsorbant obtenue dans l'exemple 1, en quantité telle, que compte tenu de l'humidité résiduelle de la poudre, on ait 92 g de polymère sec.

### Séquences b/c)

On introduit dans le réacteur une quantité d'eau telle que, compte tenu de l'humidité introduite par le superabsorbant, on ait 138 g d'eau dans le réacteur (teneur en eau du polymère superabsorbant gonflé en suspension dans le milieu heptane égale à 60% en poids du total polymère + eau). La température du fluide caloporteur est consignée à 70°C.

### Séquence d)

Pendant le déroulement de la séquence précédente, on procède dans un réacteur équipé comme celui de la séquence a, à la neutralisation de 92 grammes de solution aqueuse à 80 % en poids d'acide acrylique par 135,59 grammes de lessive de soude à 22,62 %, en procédant suffisamment lentement pour ne pas dépasser la température de 30°C. A cette solution d'acide acrylique partiellement neutralisé (75 moles %), on ajoute 2 grammes de carboxyméthylcellulose et 0,46 gramme d'une solution aqueuse à 10 % de nonylphénol éthoxylé à 50 moles d'oxyde d'éthylène (Remcopal 31250 de CECA S.A.), puis 2,76 grammes d'une solution aqueuse à 2 % en poids de persulfate de potassium et 7 grammes d'une solution aqueuse à 2 % d'éthylèneglycoldiglycidyléther.

### Séquence e)

La charge de monomère préparée dans la séquence d ci-dessus est introduite goutte à goutte dans le réacteur tel qu'il est en fin de séquence c. Le temps d'introduction est d'environ 30 minutes, la température du fluide de la double enveloppe restant consignée à 70°C, sous barbotage d'azote, la vitesse d'agitation étant maintenue à 400-600 tours / minute. Du fait de l'introduction de cette charge, relativement froide, la température du réacteur descend légèrement, sans toutefois jamais tomber en dessous de 65°C, puis remonte ensuite à la fois du fait du chauffage par la double enveloppe à 70°C et de l'exothermicité de la réaction de polymérisation qui redémarre bientôt. On augmente la vitesse d'agitation à 800 tours/minute et on maintient le réacteur dans ces conditions pendant une vingtaine de minutes.

### Séquence f) finale

On monte maintenant la température de consigne à 115-130°C pour éliminer l'eau et l'heptane. Après évaporation complète, on obtient un polymère superabsorbant sous forme d'une poudre qui sous le microscope apparaît comme formée d'agglomérats de billes de polymère. La fraction de poudre passant au tamis de 100 µm est inférieure à 1 %. L'absorption d'eau salée est de 60 g/g de polymère.

### Exemple 3 : procédé en deux étapes, polymérisation et agglomération selon l'invention.

On commence par dérouler les étapes a, b, et c comme dans l'exemple 1.

On enchaîne ensuite les séquences d et e comme dans l'exemple 2, et on termine l'opération par une séquence finale f, également comme dans l'exemple 2.

On obtient ainsi une poudre de polymère superabsorbant sous forme d'une poudre d'agglomérats de billes, dont le passant à 100 micromètres est inférieur à 1 %, et dont l'absorption d'eau salée est de 62 g/g de polymère.

### Exemple 4 : compositions aqueuses selon l'invention, et contrôle de la température selon Sumitomo

On procède comme dans l'exemple 3, aux différences près suivantes.

L'étape de polymérisation du monomère en suspension inverse aboutissant à l'obtention d'une suspension de billes individuelles de monomère dans le solvant hydrocarboné est réalisée par exécution des séquences a, b, et c, comme dans l'exemple 1, mais à la fin de la séquence c, la température de consigne du réacteur est affichée à 40°C de façon à amener le contenu du réacteur à cette température. Lorsque cette température est atteinte, le contrôle thermique de la double enveloppe est inactivé. On engage alors la séquence suivante.

### Séquence d)

Pendant le déroulement de la séquence précédente, on procède dans un réacteur équipé comme celui de la séquence a, à la neutralisation de 92 grammes de solution aqueuse à 80 % en poids d'acide acrylique par 135,59 grammes de lessive de soude à 22,62 %, en procédant suffisamment lentement pour ne pas dépasser la température de 30°C. A cette solution d'acide acrylique partiellement neutralisé (75 moles %), on ajoute 2 grammes de carboxyméthylcellulose et 0,46 gramme d'une solution aqueuse à 10 % de nonylphénol éthoxylé à 50 moles d'oxyde d'éthylène (Remcopal 31250 de CECA S.A.), puis 2,76 grammes d'une solution aqueuse à 2 % en poids de persulfate de potassium et 2 grammes d'une solution aqueuse à 2 % d'éthylèneglycoldiglycidyléther.

### Séquence e)

On introduit goutte à goutte dans le réacteur, sous barbotage d'azote, la vitesse d'agitation étant maintenue à 400-600 tours / minute, la charge aqueuse préparée selon la séquence c. La température du contenu du réacteur s'abaisse encore jusque vers 35°C. On maintient ces conditions pendant 15 minutes environ pour stabilisation, puis on remet en route le contrôle thermique du réacteur en consignant la température du fluide caloporteur à 70°C. La polymérisation démarre. On maintient ces nouvelles conditions pendant une vingtaine de minutes à partir du moment où la température du milieu a atteint 70°C.

### Séquence f) finale

Comme dans l'exemple 1.

On obtient ainsi une poudre sensiblement identique à celle que l'on a obtenue dans l'exemple 3, au prix cependant d'une plus grande complication expérimentale, d'un plus long délai de réalisation et d'une consommation sensiblement plus importante dans les cycles de refroidissement / réchauffage des séquences c et f.

### Exemple 5 : compositions aqueuses selon Sumitomo, et contrôle de la température selon l'invention

On procède comme dans l'exemple 4, aux différences près suivantes.

### Séquence a)

Dans le dispositif décrit à l'exemple 1, on introduit 275 g d'heptane que l'on porte à 80°C et on y dissout sous agitation de 400 tours/minute, 0,74 g de di/tristéarate de saccharose et 1,07 g de polyéthylène modifié par l'anhydride maléique.

### Séquence b)

A part, on neutralise 92 g d'une solution aqueuse à 80 % en poids d'acide acrylique par 135,59g de lessive de soude à 22 %. On ajoute 1g de carboxyméthylcellulose, puis 2,76 g d'une solution aqueuse à 2 % de persulfate de potassium, et 0,92 g d'une solution aqueuse à 2 % d'éthylèneglycoldiglycidyléther.

### Séquence c)

Le réacteur étant maintenu sous agitation de 400 tours/minute et sous balayage d'azote à raison de 80 litres/minute, on y introduit peu à peu la phase aqueuse précédemment préparée qui passe en suspension inverse dans l'heptane. La température est portée à 70°C pour provoquer la polymérisation ; elle est maintenue à ce niveau pendant 30 minutes. La température est alors ramenée à 45°C.

### Séquence d)

Pendant le déroulement de l'opération précédente, on procède à part à la neutralisation de 92 g de solution aqueuse à 80 % en poids d'acide acrylique par 135,59g de lessive de soude à 22,6 %, puis on ajoute 2,76g d'une solution aqueuse à 2 % de persulfate de potassium et 0,92 g d'une solution aqueuse à 2 % d'éthylène glycol diglycidyléther. Cette phase aqueuse qui constitue la charge II de monomère est alors ramenée à 10°C.

### Séquence e)

L'agitation dans le réacteur est portée à 800 tours/minutes, le balayage d'azote étant maintenu à 80 litreslminutes. On y introduit peu à peu la charge d. La charge d étant introduite, on laisse l'absorption se poursuivre pendant 5 minutes à la température de 45°C. Après quoi la température est portée à 70°C pour engager la seconde phase de polymérisation. On laisse la polymérisation se poursuivre pendant 30 minutes.

### Séquence f) finale

On élimine l'heptane et la majeure partie de l'eau par distillation.

Le produit final est une poudre dont la granulométrie moyenne est de 150 µm, dont le passant à 100 µm est inacceptable avec une valeur supérieure à 15 %, et dont l'absorption d'eau salée à 0,9 % est de 55 g/g.

### Exemple 6

Dans cet exemple, on exploite totalement la faculté de mener l'opération à haute température. C'est une variante intéressante de l'exemple précédent.

### Séquence a)

On procède comme dans l'exemple 1, à la différence près qu'en fin de séquence, on ramène la température à 70°C en fixant la température du fluide caloporteur de la double enveloppe du réacteur à cette température.

### Séquence b)

On prépare une solution aqueuse de monomère comme dans l'exemple 1.

### Séquence c)

Le réacteur avec la solution à base d'heptane étant maintenu sous agitation de 400 tours/minute et sous balayage d'azote, on y introduit rapidement la phase aqueuse précédemment préparée qui passe en suspension inverse dans l'heptane. L'introduction de la phase aqueuse dans le réacteur provoque une chute momentanée de la température du milieu, vite compensée par le fait que la température de la double enveloppe est consignée à 70°C. La polymérisation s'engage, dont l'exothermicité peut amener le milieu à une température pl4s élevée, ramenée à 70°C par le même mécanisme dès que l'exothermie de polymérisation cesse. Il se forme une suspension de polymère, qui se maintient automatiquement à 70°C.

### Séquence d)

Pendant le déroulement de l'opération précédente, on procède à la préparation d'une nouvelle charge de solution aqueuse de monomère, comme il est décrit à la séquence d de l'exemple 3.

### Séquences e) et f)

Elles sont conduites comme les séquences e et f de l'exemple 3.

On obtient ainsi une poudre de polymère superabsorbant sous forme d'une poudre d'agglomérats de billes, dont le passant à 100 micromètres est inférieur à 1%. Son absorption d'eau salée est de 65 g/g.

### Exemple 7 :contre exemples partiels

Toutes les séquences se déroulent comme dans l'exemple précédent, à l'exception de la séquence d, dans laquelle on prépare la solution aqueuse de monomère en omettant l'addition de l'additif hydrophile, c'est-à-dire de la carboxyméthylcellulose et du nonylphénol éthoxylé à 50 moles d'oxyde d'éthylène.

On obtient ainsi une poudre de polymère superabsorbant constitué essentiellement de billes avec un passant à 100 µm inacceptable de 36 %.

Si on omet le nonylphénol éthoxylé à 50 moles d'oxyde d'éthylène, on sort une poudre contenant 30 % de fines. Si c'est l'épaississant qui est omis, la poudre obtenue contient 10 % de fines.

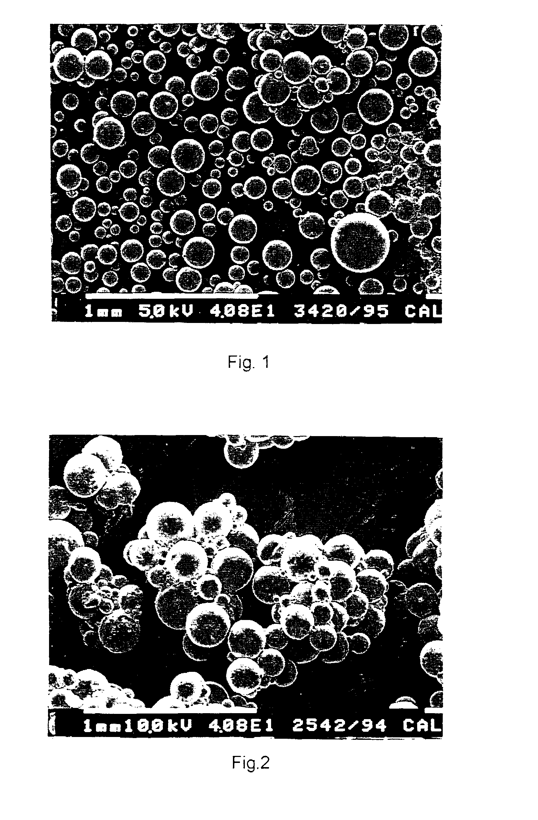

## Revendications

1. Procédé d'obtention de polymère acrylique superabsorbant sous la forme d'une poudre d'agglomérats de particules sphériques par agglomération de particules fines de polymère superabsorbant à l'aide d'un agglomérant constitué par une solution aqueuse de monomère *insaturé hydrosoluble,* en suspension dans un milieu liquide hydrocarboné non-solvant pour le polymère et pour l'agglomérant, avec la condition que soient respecté l'ensemble des conditions suivantes :
- la solution aqueuse de monomère est introduite dans le milieu à la température de polymérisation du monomère, généralement *comprise entre 60 et 75°C,*
- la quantité de monomère agglomérant est comprise entre 50 % et 200 % en poids du polymère à agglomérer,
- la solution aqueuse de monomère contient en outre un additif hydrophile constitué d'un épaississant et d'un tensioactif d'HLB supérieur ou égal à 8 *ainsi qu'un* amorceur de polymérisation.

2. Procédé selon la revendication 1, caractérisé en ce que la suspension de polymère superabsorbant à agglomérer est réalisée par mise en suspension d'une poudre de polymère superabsorbant dans un liquide hydrocarboné dans lequel le polymère n'est pas soluble.

3. Procédé selon la revendication 2, caractérisé en ce que préalablement à l'agglomération, le polymère superabsorbant mis en suspension dans un liquide hydrocarboné dans lequel il n'est pas soluble est gonflé par de l'eau.

4. Procédé selon la revendication 1, caractérisé en ce que la suspension de polymère superabsorbant à agglomérer est une suspension telle qu'elle est obtenue par polymérisation d'une solution aqueuse de monomère en suspension inverse dans un liquide hydrocarboné dans lequel le polymère et le monomère d'agglomération ne sont pas solubles.

5. Procédé selon la revendication 1, caractérisé en ce que l'épaississant qui est l'un des composants de l'agent hydrophile est pris dans le groupe constitué par les celluloses hydrosolubles modifiées et les polymères et copolymères acryliques hydrosolubles.

6. Procédé selon la revendication 1, caractérisé en ce que le tensioactif qui est l'un des composants de l'agent hydrophile est pris dans le groupe constitué par les alkylphénols éthoxylés, le sorbitane éthoxylé ou les dérivés de sorbitol éthoxylés qui contiennent 10 à 100 molécules d'oxyde d'éthylène par molécule.

7. Procédé selon la revendication 1, caractérisé en ce que l'agent hydrophile contient comme agent épaississant de la carboxyméthylcellulose et comme tensioactif hydroxylé le nonylphénol éthoxylé à 50 moles d'oxyde d'éthylène.

## Patentansprüche

1. Verfahren zur Herstellung eines hochabsorbierenden Acrylpolymeren in Form eines Pulvers aus Agglomeraten kugelförmiger Teilchen durch Agglomerieren von feinen Teilchen eines hochabsorbierenden Polymeren mit Hilfe eines Bindemittels, das aus einer wäßrigen Lösung eines ungesättigten wasserlöslichen Monomeren besteht, in Suspension in einem flüssigen kohlenwasserstoffhaltigen das Polymere und das Bindemittel nicht lösenden Medium mit der Maßgabe, daß alle folgenden Bedingungen eingehalten sind:
- die wäßrige Lösung des Monomeren wird dem Medium bei der Polymerisationstemperatur des Monomeren, im allgemeinen zwischen 60 und 75 °C, zugegeben,
- die Menge des bindenden Monomeren liegt zwischen 50 Gew.-% und 200 Gew.-% des zu agglomerierenden Polymeren,
- die wäßrige Lösung des Monomeren enthält darüber hinaus ein hydrophiles Additiv, das aus einem Verdickungsmittel und einem Tensid mit einem HLB-Wert größer oder gleich 8 sowie einem Polymerisations-starter.

2. Verfahren nach Anspruch 1, dadurch gekennzeichnet, daß die Suspension des hochabsorbierenden zu agglomerierenden Polymeren durch Suspendieren eines Pulvers eines hochabsorbierenden Polymeren in einer kohlenwasserstoffhaltigen Flüssigkeit erfolgt, in der das Polymere nicht löslich ist.

3. Verfahren nach Anspruch 2, dadurch gekennzeichnet, daß vor dem Agglomerieren das hochabsorbierende Polymere, das in einer kohlenwasserstoffhaltigen Flüssigkeit suspendiert ist, in der es nicht löslich ist, mit Wasser aufquellen wird.

4. Verfahren nach Anspruch 1, dadurch gekennzeichnet, daß die Suspension des hochabsorbierenden zu agglomerierenden Polymeren eine Suspension ist, wie sie durch Polymerisation einer wäßrigen Lösung eines Monomeren in inverser Suspension in einer kohlenwasserstoffhaltigen Flüssigkeit, in der das Polymere und das Monomere der Agglomerierung nicht löslich sind, erhalten wird.

5. Verfahren nach Anspruch 1, dadurch gekennzeichnet, daß das Verdickungsmittel, das einer der Bestandteile des hydrophilen Mittels ist, zur Gruppe der wasserlöslichen modifizierten Cellulose und den wasserlöslichen Acrylpolymeren und -copolymeren gehört.

6. Verfahren nach Anspruch 1, dadurch gekennzeichnet, daß das Tensid, das einer der Bestandteile des hydrophilen Mittels ist, zur Gruppe der ethoxylierten Acrylphenole, des ethoxylierten Sorbitans oder den ethoxylierten Sorbitderivaten gehört, die 10 bis 100 Ethylenoxidmoleküle pro Molekül enthalten.

7. Verfahren nach Anspruch 1, dadurch gekennzeichnet, daß das hydrophile Mittel als Verdickungsmittel Carboxymethylcellulose und als hydroxyliertes Tensid ethoxiliertes Nonylphenol mit 50 Mol Ethylenoxyd enthält.

## Claims

1. Process for obtaining a superabsorbent acrylic polymer in the form of a powder of agglomerates comprising spherical particles by agglomerating fine particles of superabsorbent polymer with the aid of an agglomerant consisting of an aqueous solution of water-soluble unsaturated monomer, in suspension in a liquid hydrocarbon medium which is a non-solvent for the polymer and for the agglomerant, with the condition that all of the following conditions be met:
- the aqueous monomer solution is introduced into the medium at the monomer polymerization temperature, generally lying between 60 and 75°C;
- the amount of agglomerating monomer lies between 50% and 200% by weight of the polymer to be agglomerated;
- the aqueous monomer solution furthermore contains a hydrophilic additive consisting of a thickener and of a surfactant with an HLB of greater than or equal to 8 as well as a polymerization initiator.

2. Process according to Claim 1, characterized in that the suspension of superabsorbent polymer to be agglomerated is produced by putting a superabsorbent polymer powder in suspension in a hydrocarbon liquid in which the polymer is not soluble.

3. Process according to Claim 2, characterized in that, prior to agglomeration, the superabsorbent polymer, put in suspension in a hydrocarbon liquid in which it is not soluble, is swollen with water.

4. Process according to Claim 1, characterized in that the suspension of superabsorbent polymer to be agglomerated is a suspension such as is obtained by inverse suspension polymerization of an aqueous monomer solution in a hydrocarbon liquid in which the polymer and the agglomerating monomer are not soluble.

5. Process according to Claim 1, characterized in that the thickener which is one of the components of the hydrophilic agent is chosen from the group consisting of water-soluble modified celluloses and water-soluble acrylic polymers and copolymers.

6. Process according to Claim 1, characterized in that the surfactant which is one of the components of the hydrophilic agent is chosen from the group consisting of ethoxylated alkylphenols, ethoxylated sorbitan or ethoxylated sorbitol derivatives which contain 10 to 100 ethylene oxide molecules per molecule.

7. Process according to Claim 1, characterized in that the hydrophilic agent contains carboxymethylcellulose as thickener and ethoxylated nonylphenol containing 50 mol of ethylene oxide as hydroxylated surfactant.
